(19) Europäisches Patentamt
European Patent Office
Office européen des brevets

(11) EP 1 368 001 B1

# (12) EUROPEAN PATENT SPECIFICATION

(45) Date of publication and mention of the grant of the patent:
**26.10.2005 Bulletin 2005/43**

(21) Application number: **02712105.2**

(22) Date of filing: **22.02.2002**

(51) Int Cl.[7]: **A61K 9/16**, A61K 31/56

(86) International application number:
**PCT/GB2002/000766**

(87) International publication number:
**WO 2002/067893 (06.09.2002 Gazette 2002/36)**

(54) **PHARMACEUTICAL FORMULATION COMPRISING BICALUTAMIDE**

PHARMAZEUTISCHE FORMULIERUNG ENTHALTEND BICALUTAMID

PREPARATION PHARMACEUTIQUE COMPRENANT DU BICALUTAMIDE

(84) Designated Contracting States:
**AT BE CH CY DE DK ES FI FR GB GR IE IT LI LU MC NL PT SE TR**
Designated Extension States:
**AL LT LV MK RO SI**

(30) Priority: **27.02.2001 GB 0104749**
**19.07.2001 SE 0102572**

(43) Date of publication of application:
**10.12.2003 Bulletin 2003/50**

(73) Proprietor: **AstraZeneca AB**
**S-151 85 Södertälje (SE)**

(72) Inventors:
- **BATEMAN, Nicola**
  **Macclesfield, Cheshire SK10 4TG (GB)**
- **CAHILL, Julie**
  **Macclesfield, CheshireSK10 4TG (GB)**

(74) Representative:
**Gainey, Laurence David Scott et al**
**AstraZeneca AB,**
**Global Intellectual Property**
**151 85 Sodartalje (SE)**

(56) References cited:
- **DENIS L ET AL: "PHARMACODYNAMICS AND PHARMACOKINETICS OF BICALUTAMIDE: DEFINING AN ACTIVE DOSING REGIMEN" UROLOGY, BELLE MEAD, NJ, US, vol. 47, no. SUPPL 1A, 1996, pages 26-28, XP000605159 ISSN: 0090-4295**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 1 368 001 B1

## Description

The present invention relates to a pharmaceutical formulation comprising bicalutamide and an enteric polymer having a $pK_a$ from 3 to 6. The invention also relates to a daily pharmaceutical dose of bicalutamide provided by such a formulation. In addition, the invention relates to the use of such an enteric polymer in solid dispersion with bicalutamide for increasing the bioavailability of the bicalutamide; for reducing inter-patient variability in plasma concentrations of bicalutamide; or for treating and/or reducing the risk of prostate cancer in a patient.

## BACKGROUND TO THE INVENTION

Bicalutamide, a non-steroidal anti-androgen, is the racemate of 4'-cyano-α',α',α'-trifluoro-3-(4-fluorophenylsulphonyl)-2-hydroxy-2-methylpropiono-*m*-toluidide and is known by the AstraZeneca trade name CASODEX™. EP-100172 discloses 4'-cyano-α',α',α'-trifluoro-3-(4-fluorophenylsulphonyl)-2-hydroxy-2-methylpropiono-*m*-toluidide (named in EP-100172 as 4-cyano-3-trifluoromethyl-*N*-(3-*p*-fluorophenylsulphonyl-2-hydroxy-2-methylpropionyl)aniline) as the 8th compound listed in the table in Example 6. The corresponding structure is shown in formula I: -

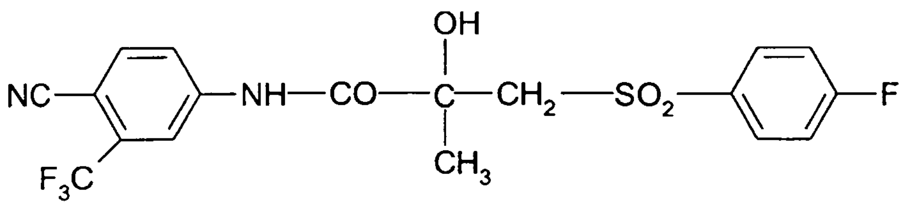

I .

Bicalutamide can be used to combat prostate cancer. The properties and usefulness of bicalutamide as an anti-androgen have been reviewed in B J A Furr *et al*., Urology, 1996, 47 (Suppl. 1A), 13-25, and G J C Kolvenbag *et al*., Urology, 1996, 47 (Suppl. 1A), 70-79.

Bicalutamide is used in conventional oral tablet form (eg, at a daily monotherapy dose of 150mg) to combat prostate cancer in men. The bioavailability of the bicalutamide to the patient is determined to a certain extent by the dissolution rate and solubility of the drug in the GI tract, which affects absorption across mucosal membranes in the GI tract. The relative bioavailability of bicalutamide for a series of formulations can be assessed by determining the area under the curve (AUC) of a graph of plasma bicalutamide concentration v. time elapsed since administration of the bicalutamide. As a consequence of sub-optimal rates of dissolution and degree of solubility of the drug, there is observed a high degree of inter-patient variability in the bioavailability of bicalutamide administered in conventional tablet form. This may result in sub-optimal treatment efficacy in a proportion of patients. In addition, the maximum systemic exposure achievable after dosing the conventional tablet is limited, such that at conventional tablet doses in excess of 150mg, there is a significant reduction in bicalutamide bioavailability. At conventional tablet doses above 300mg, no further significant increase in systemic exposure is achievable

It would be desirable to extend the therapeutic potential of bicalutamide by increasing the bioavailability of the drug and/or reducing inter-patient variability in plasma concentrations of bicalutamide as a result of reduced inter-patient variability in the absorption of bicalutamide.

Such increased bioavailability could be useful in enabling a reduction in the daily dose of bicalutamide required to achieve the same level of bioavailability seen with a conventional formulation.

A possible benefit of achieving relatively higher bioavailability could also be the ability to extend treatment to more advanced stages of prostate cancer than are currently treated with the conventional formulations. This could be useful, for example, for treating patients with metastatic prostate cancer, using for example bicalutamide as a monotherapy (ie, not in combination with LHRH analogue therapy or surgical castration).

As another advantage, it would also be desirable to reduce inter-patient variability in plasma concentrations of bicalutamide as a result of reduced inter-patient variability in the absorption of bicalutamide. This would increase predictability of the treatment and increase uniformity of treatment in a patient population.

EP-0988863 deals with the issue of increasing the bioavailability of poorly soluble drugs in general. Bicalutamide is not specifically addressed. The disclosed solution is to provide a formulation comprising a water-insoluble

complex of the drug and a water-insoluble ionic polymer. No specific class of polymer is required, and the polymer can be cationic or anionic, but must have a molecular weight greater than about 80,000 D and a glass transition temperature equal or greater than about 50°C.

EP-1027886 also deals with the issue of increasing the bioavailability of poorly soluble drugs in general. Again, bicalutamide is not specifically addressed. The disclosed solution is to provide a solid dispersion formulation comprising a low-solubility drug and a polymer. The latter can be one of many possible polymers, as long as it has a glass transition temperature of at least 100°C measured at 50% relative humidity. Some enteric polymers (eg, HPMCP polymers, including grades HP-50™ , HP-55™ and HP-55S™) are explicitly excluded from use, since it is explained that all of these polymers absorb sufficient water upon equilibration at 50% relative humidity that their respective glass transition temperatures drop below 100°C. Hydroxypropyl methylcellulose acetate succinate (HPMCAS), another enteric polymer, is also excluded when used alone.

The present invention aims to improve upon the conventional formulation of bicalutamide by increasing the therapeutic potential of bicalutamide as discussed above.

## SUMMARY OF THE INVENTION

The present invention fulfils this aim by providing a pharmaceutical formulation for mucosal administration to a patient, the formulation comprising bicalutamide in solid dispersion with an enteric polymer having a $pK_a$ from 3 to 6. It is contemplated that one or a mixture of such polymers can be used.

The invention also provides a daily pharmaceutical dose of bicalutamide mucosally administrable to a patient for treating and/or reducing the risk of prostate cancer in the patient, wherein the dose comprises from 25 to 1000 mg of bicalutamide in a solid dispersion with an enteric polymer having a $pK_a$ from 3 to 6.

Further aspects of the invention relate to the use of an enteric polymer having a $pK_a$ from 3 to 6 in solid dispersion with bicalutamide, in the manufacture of a medicament mucosally administrable to a patient, for

(a) increasing the bioavailability of bicalutamide in the patient; or

(b) treating and/or reducing the risk of prostate cancer in the patient. As explained below, reducing the risk of prostate cancer includes reducing the risk of re-occurrence of prostate cancer.

In addition, the invention relates to the use of an enteric polymer having a $pK_a$ from 3 to 6 in solid dispersion with bicalutamide, in the manufacture of a medicament mucosally administrable to patients, for reducing inter-patient variability in plasma concentrations of bicalutamide.

## FIGURES

Fig. 1 Dissolution of bicalutamide from various solid dispersion formulations comprising enteric polymers (50mg bicalutamide in 900ml of media).

Key:-

| | |
|---|---|
| Circles - | conventional bicalutamide tablet formulation |
| Broken line - | HPMCP HP-55S |
| Diamonds - | EUDRAGIT™ L100 |
| Squares - | HPMCAS AQOAT™ LG |

Fig. 2 Dissolution of bicalutamide from various solid dispersion formulations comprising enteric or non-enteric polymers (50mg bicalutamide in 900ml of media).

Key:-

| | |
|---|---|
| Diamonds - | HPMCP PHARMACOAT™ 606 |
| Squares - | METOLOSE™ 60SH 50cp |
| Triangles - | PEG4000 |
| Crosses - | PLA:PEG [2kD:2kD] |
| Broken line - | HPMCP HP-55S |
| Circles - | conventional bicalutamide tablet formulation |

Fig. 3 Dissolution of bicalutamide from solid dispersion formulations (50mg bicalutamide in 900ml of media)

comprising bicalutamide with HP-55S at various weight ratios.

Key:-

The following ratios relate to weight ratios of bicalutamide:HP-55S

| | |
|---|---|
| Diamonds - | 1:5 |
| Squares - | 1:4 |
| Triangles - | 1:3 |
| Crosses - | 1:2 |
| Circles - | 1:1 |
| Broken line - | conventional bicalutamide tablet formulation. |

Fig. 4 Plasma profiles following administration of bicalutamide formulations to dogs (n=6, 450mg bicalutamide dose). The vertical bars indicate variability.

Key:-

| | |
|---|---|
| Solid line - | solid dispersion of 1:3 by weight of bicalutamide:HP-55S |
| Broken line - | conventional bicalutamide tablet formulation. |

## DETAILED DESCRIPTION OF THE INVENTION

The inventors chose to investigate solid dispersion formulations as a possible means of increasing the therapeutic potential of bicalutamide. The aim was to increase the therapeutic potential by achieving one or both of an in increase the bioavailability of bicalutamide and a decrease in inter-patient variability in plasma concentrations of bicalutamide.

The prior art teaches a very wide range of possible polymers for solid dispersion, in order to increase the bioavailability of drugs in general. The inventors have now surprisingly found that the therapeutic potential of bicalutamide can be increased by formulating bicalutamide in a solid dispersion specifically with an enteric polymer having a $pK_a$ from 3 to 6. As the non-limiting example section below demonstrates, such an increase in therapeutic potential for bicalutamide is not achieved with other polymers.

Various materials have conventionally been used to coat pharmaceutical tablets, capsules and granules to be compressed into tablets or used to fill capsules. Reference is made to Schroeter, L C, Coating of Tablets, Capsules and Pills, Remington's Pharmaceutical Sciences, 13$^{th}$ ed., 1965, p. 604, which reviews more than 60 enteric coating materials. These include coating materials (eg, camauba wax, stearic acid and paraffin) that rely on erosion in the intestinal tract, and enteric polymers that are designed to resist the destructive action of gastric fluid and to disintegrate in the intestinal tract. Enteric polymers are thus by definition pH-sensitive and have ionisable acid groups. Ionisation, and thus increased solubility, occurs in the intestinal tract, so that the polymers are substantially insoluble at the pH of gastric fluid (pH 1 to 3.5), but dissolve at the pH of intestinal fluid. The particular enteric polymers used in the present invention are those enteric polymers that have a $pK_a$ from 3 to 6. In one example, the lower end of this range is 3.5, 4 or 4.5. In one example, the upper end of the range is 5 or 5.5.

As the skilled addressee knows, the Henderson-Hasselbach equation may be used to determine the $pK_a$ according to the following equation:-

$$pK_a = pH - \log (\text{concentration of non-ionised polymer} \div \text{concentration of ionised polymer})$$

At a pH two units below the $pK_a$, only approximately 1% of the acid groups will be ionised, and the polymer will be poorly soluble in gastric fluid. As the pH increases, the percentage of ionised acid groups increases, such that when the pH exceeds the $pK_a$ by two units the percentage of ionised groups is approximately 100%, and the polymer will be soluble in the intestines.

In one embodiment, the enteric polymer is selected from hydroxypropyl methylcellulose acetate succinate (HPMCAS), hydroxpropyl methylcellulose acetate pthalate, hydroxypropyl methylcellulose acetate, hydroxypropyl methylcellulose succinate, a methacrylic acid copolymer, polyvinyl acetate phthalate (PVAP), cellulose acetate phthalate (CAP), methylcellulose acetate phthalate, ethyl cellulose acetate phthalate, hydroxypropyl cellulose acetate phthalate, hydroxypropyl methylcellulose phthalate (HPMCP), cellulose proprionate pthalate, hydroxypropyl cellulose butyrate pthalate, hydroxypropyl cellulose acetate pthalate succinate, hydroxypropyl methylcellulose trimellitate, cellulose acetate trimellitate (CAT), methylcellulose acetate trimellitate, ethyl cellulose acetate trimellitate, hydroxypropyl cellulose acetate trimellitate, hydroxypropyl methylcellulose acetate trimellitate, hydroxypropyl cellulose acetate trimellitate succinate, cellulose proprionate trimellitate, cellulose butyrate trimellitate, cellulose acetate terepthalate and cellulose

actetate isopthalate.

The use of the term "hydroxypropyl methylcellulose phthalate polymer", or HPMCP, is known to the skilled reader for classifying a group of polymers which share the same basic structural features and include such polymers as: hypromellose phthalate; methylhydroxypropylcellulosi pthalas; cellulose, hydrogen 1,2-benzenedicarboxylate, 2-hydroxypropyl methyl; as well as commercially available polymers HP-55™, HP-55S™ and HP-50™ (available from Shin-Etsu Chemical Industry Co., Ltd., Japan or appointed distributors).

Preferably the hydroxypropylmethylcellulose phthalate polymer has a molecular weight (Mw) from 20kDa to 200kDa, eg from 80kDa to 130kDa. In one embodiment, the Mw is less than 150kDa, or less than 100kDa. HP-50, HP-55 and HP-55S are polymers known in the literature and widely used as an enteric coating for oral formulations. HP-55 has a Mw 84kDa. HP-55S has a Mw of 132kDa. HP-50 has a Mw 78kDa. HP-50 is soluble at pH≥5, whereas HP-55 and HP-55S are soluble at pH≥5.5. In one embodiment, the bicalutamide is in a solid dispersion with at least one polymer selected from HP-50, HP-55 and HP-55S. Thus, it is contemplated that a mixture of two or more of these HPMCP polymers can be used.

HPMCAS (trade name: AQOAT, available from Shin-Etsu Chemical Industry Co., Ltd., Japan or appointed distributors) is available in the following grades: AS-LF, AS-MF, AS-HF, AS-LG, AS-MG and AS-HG. The AS-L grades are soluble at pH ≥5.5, the AS-M grades are soluble at pH≥ 6.0 and the AS-H grades are soluble at pH≥ 6.5. In one embodiment, the bicalutamide is in a solid dispersion with at least one polymer selected from HPMCAS grades AS-L, AS-M, AS-H. Thus, it is contemplated that a mixture of two or more of these HPMCAS polymers can be used.

Methacrylic acid copolymer is a fully polymerised copolymer of methacrylic acid and methacrylic acid methyl ester. Grade A (trade name: EUDRAGIT™ L 100, available from Rohm Pharma or appointed distributors) and grade B (trade name EUDRAGIT™ S 100) are available. The grades differ in the ratio of free carboxyl groups to ester groups and, therefore, differ in solubility profiles. Type A has a ratio of approximately 1:1 and is soluble at pH≥6. Type B has a ratio of approximately 1:2 and is soluble at pH≥7. Another grade (EUDRAGIT™ L 30 D-55) is soluble at pH≥5.5. In one embodiment, the bicalutamide is in a solid dispersion with at least one methacrylic acid copolymer. Thus, it is contemplated that a mixture of two or more of these polymers (eg, grades A and B) can be used.

PVAP is soluble at pH≥5 and is available from Colorcon Inc or appointed distributors.

CAP (available from FMC Corporation as part of a powdered product, AQUATERIC™) solubilises at pH≥6.5.

CAT is available from Eastman Fine chemicals, Zurich, Switzerland.

A preferred ratio of bicalutamide : enteric polymer by weight is from 1:0.25 to 1:10. More preferably the lower limit of this range is 1:0.5, 1:0.75 or 1:1. Preferably, the upper limit of this range is 1:3 or 1:5. A most preferred range of ratios is 1:1 to 1:3.

One aspect of the invention provides a daily pharmaceutical dose of bicalutamide mucosally administrable to a patient for treating and/or reducing the risk of prostate cancer in the patient, wherein the dose comprises 25 to 1000mg of bicalutamide in a solid dispersion with an enteric polymer having a $pK_a$ from 3 to 6. Preferably, the dose comprises an upper limit of 900, 800, 750, 600, 500, 450, 400, 300, 200, 150, 125, 100, 75 or 50mg of bicalutamide. In one example, the dose comprises 150 or 450mg of bicalutamide.

Additional excipients may be included in the formulation or dose. For example, the formulation or dose may comprise one or more fillers, binder, disintegrants and/or lubricants.

Suitable fillers include, for example, lactose, sugar, starches, modified starches, mannitol, sorbitol, inorganic salts, cellulose derivatives (e.g. microcrystalline cellulose, cellulose), calcium sulphate, xylitol and lactitol.

Suitable binders include, for example, polyvinylpyrrolidone, lactose, starches, modified starches, sugars, gum acacia, gum tragacanth, guar gum, pectin, wax binders, microcrystalline cellulose, methylcellulose, carboxymethylcellulose, hydroxypropyl methylcellulose, hydroxyethyl cellulose, hydroxypropyl cellulose, copolyvidone, gelatin and sodium alginate.

Suitable disintegrants include, for example, crosscarmellose sodium, crospovidone, polyvinylpyrrolidone, sodium starch glycollate, corn starch, microcrystalline cellulose, hydroxypropyl methylcellulose and hydroxypropyl cellulose.

Suitable lubricants include, for example, magnesium stearate, stearic acid, palmitic acid, calcium stearate, talc, camauba wax, hydrogenated vegetable oils, mineral oil, polyethylene glycols and sodium stearyl fumarate.

Additional conventional excipients which may be added include preservatives, stabilisers, anti-oxidants, silica flow conditioners, antiadherents or glidants.

Other suitable fillers, binders, disintegrants, lubricants and additional excipients which may be used are described in the Handbook of Pharmaceutical Excipients, 3rd Edition; The Theory and Practice of Industrial Pharmacy, 3rd Edition 1986; Pharmaceutical Dosage Forms 1998; Modem Pharmaceutics, 3rd Edition 1995; Remington's Pharmaceutical Sciences 20th Edition 2000.

Preferably, the bicalutamide will be present in an amount of 1 to 80% , and preferably from 1 to 50% (more preferably 2 to 25% or 2 to 15%) by weight of the solid dispersion.

Preferably, one or more fillers will be present in an amount of 1 to 70% by weight of the formulation or dose.

Preferably, one or more binders will be present in an amount of 2 to 40% by weight of the formulation or dose.
Preferably, one or more disintegrants will be present in an amount of 1 to 10%, and especially 4 to 6% by weight of the formulation or dose.
It will be appreciated that a particular excipient may act as both a binder and a filler, or as a binder, a filler and a disintegrant. Typically the combined amount of filler, binder and disintegrant comprises, for example, 1 to 90% by weight of the formulation or dose.
Preferably, one or more lubricants will be present in an amount of 0.5 to 3%, and especially 1 to 2% by weight of the formulation or dose.
Preferably, one or more wetting agents will be present in the solid dispersion in an amount of 0.1 to 5% (more preferably, 1 to 2%) by weight of the solid dispersion. The presence of a wetting agent provides a further enhancement of the increase in therapeutic potential achieved with the present invention. Examples of suitable wetting agents include sodium dodecyl sulphate (sodium lauryl sulphate); docusate sodium; polyoxyethylen sorbitan fatty acid esters, eg polysorbates 20, 40, 60 and 80; polyoxyethylene castor oil derivatives, eg Cremophor RH40™ ; and poloxamers.
Methods for preparing solid dispersions are known in the art and typically comprise the steps of dissolving the drug and the polymer in a common solvent and evaporating the solvent. The solvent can be routinely selected according to the polymer used and the preparation method. Examples of solvents are: acetone, acetone/dichloromethane, methanol/dichloromethane, acetone/water, acetone/ethanol, dichloromethane/ethanol or ethanol/water. For HP-50, for example, the last four solvents can be used. For HPMCAS, for example, acetone, methanol, ethanol/water and methylene chloride/ethanol can be used. For methacrylic acid copolymers, isopropyl alcohol can be used. For polyvninly acetate phthalate, for example, methanol, ethanol, acetone/methanol, acetone/ethanol and methanol/methylene chloride can be used. For CAP, for example, ether/alcohols, ketones (eg, acetone), esters and cyclic ethers can be used. Methods for evaporating solvent include rotary evaporation, spray drying, lyophilisation and thin film evaporation. Other techniques may be used such as solvent controlled precipitation, pH controlled precipitation, spray congealing and supercritical fluid technology (eg, the Solution Enhanced Dispersion By Supercritical Fluid (SEDS) technique).
When referring to a solid dispersion we do not exclude the possibility that a proportion of the bicalutamide may be dissolved within the polymer used, the exact proportion, if any, will depend upon the particular enteric polymer (s) selected.
In the formulations of the invention, at least some of the bicalutamide may be present in amorphous form in the solid dispersion with the enteric polymer. The provision of the bicalutamide in amorphous form is additionally advantageous, since it further increases the solubility and dissolution rate of the bicalutamide, thereby enhancing the increase in therapeutic potential achieved with the present invention. Whether or not drug is present in amorphous form can be determined by conventional thermal analysis. In one embodiment, at least 25% of the bicalutamide in the formulation is present in amorphous form. More preferably, this amount is at least 30%, 40%, 50%, 75%, 90%, 95% or 99%.
The most preferred embodiment is where 100% of the bicalutamide in the formulation is in amorphous form.
The formulations and doses are mucosally administrable, ie administrable to mucosal membranes for absorption across the membranes. To this end, suitable routes of administration include administration by inhalation, as well as oral, intranasal and rectal administration. Oral administration is particularly preferred. A tablet or other form of the formulation would be chosen by the skilled addressee according to the route of administration.
The bicalutamide is useful to provide an anti-androgenic effect, in that this compound blocks androgen activity in a patient. The anti-androgenic effect is useful for treating cancer, for example prostate cancer. Particular examples are advanced prostate cancer and early prostate cancer. The anti-androgenic effect may be useful for prophylaxis, in order to reduce the risk of prostate cancer occurrence in patients or re-occurrence (eg, following prostatectomy or radiation therapy aimed at curing the patient). This could be especially useful in men genetically pre-disposed to prostate cancer. Conventional methods are available to classify patients according to their risk of contracting prostate cancer, for example by assessment of family history and measurements over time of particular blood proteins such as prostate specific antigen (PSA). Other uses for the anti-androgenic effect are the treatment of a non-malignant disease of the prostate gland (eg, benign prostatic hyperplasia or hypertrophy) and acne.
The patient can be a human male, eg an adult, but the treatment of other mammals is also contemplated.

## EXPERIMENTAL

### *In Vitro* Assessment of Various Solid Dispersion Formulations

The inventors formulated a solid dispersion of bicalutamide with representative enteric polymers having a $pK_a$ in the range of 3 to 6 (in this case HPMCP HP-55S, EUDRAGIT L100 and HPMCAS AQOAT LG) and compared these against a conventional bicalutamide tablet formulation and also (using HPMCP HP-55S as a representative enteric polymer) against solid dispersions using several different non-enteric polymers (polyethylene glycol (PEG) 4000, PLA:

PEG [2kD:2kD] (polylactide:methoxypolyethylene glycol [2kD:2kD]), hydroxypropyl methylcellulose (HPMC) PHARMACOAT™ 606 and METOLOSE 60SH 50cp) with bicalutamide. Each formulation had a weight ratio of bicalutamide: polymer of 1:5. The formulations were assessed for an improvement in therapeutic potential using an *in vitro* dissolution test.

The performance of solid dispersions having varying weight ratios of bicalutamide:HP-55S was also assessed.

Preparation of Solid Dispersion Formulations

Solid dispersions having a 1:5 ratio by weight of bicalutamide:polymer were prepared as follows.

0.5g of bicalutamide and 2.5g of polymer were weighed directly into a 250ml round bottom flask and dissolved in 80ml of acetone:dichloromethane (3:1). The solvent was removed on a rotary evaporator. The formulation was placed in a vacuum oven and dried under high vacuum at 40°C for 24 hours.

The formulation was retrieved from the flask and dry milled using a Fritsch mill. The formulation was then dried for a further 24 hours under high vacuum at 40°C.

In order to produce formulations having ratios other than 1:5, weights and volumes in the process should be adjusted so that they are pro-rata to those described above.

*In vitro* Dissolution Test

(a) Solid Dispersion with Enteric Polymers *v*. Solid Dispersion With Non-Enteric Polymers

The formulations were weighed into hard gelatin capsules (equivalent to 50mg drug) and dissoluted in 900ml media [either 0.25% sodium dodecyl sulphate solution or pH6.5 buffer] for one hour at 37°C (paddle speed 75rpm). 5ml samples were then removed with a plastic syringe at 5, 10, 20, 30, 45 and 60 minutes. Each sample was centrifuged (14,000rpm) at ambient temperature for 15 minutes and then analysed by HPLC using the following conditions:-

| | |
|---|---|
| Eluent | 58% ACN/42% water/0.2% formic acid |
| Column | 15cm Luna 5um, 3mm id column (with guard) |
| Detection wavelength | 270nm |
| Flow rate | 1 ml/min |
| Temperature | ambient |
| Injection | 10ul |
| Retention time | approximately 2 minutes |

Figures 1 and 2 show the results of *in vitro* dissolution tests performed on the various solid dispersions. As Fig. 1 shows, 100% of bicalutamide in solution was achieved with the HPMCP HP-55S, EUDRAGIT L100 and HPMCAS AQOAT LG solid dispersions and supersaturation was maintained over the 60 minute test (ie, no drug precipitation was observed), which is a significant improvement over the conventional tablet. Compare this against the results (Fig. 2) for the PLA:PEG solid dispersion, which did not show any improvement over the conventional tablet formulation. The PEG4000 solid dispersion also was much inferior to the formulations using enteric polymers (Fig. 2), the former achieving only just over 40% of bicalutamide in solution. In addition, reference to Fig. 2 shows that the solid dispersions with METOLOSE 60SH 50cp and HPMC PHARMACOAT 606 only achieved approximately 58% and 70% of bicalutamide in solution.

(b) Solid Dispersions With Varying Ratios of Bicalutamide:HP-55S

Solid dispersions were made with weight ratios of 1:1, 1:2, 1:3, 1:4 and 1:5 bicalutamide:HP-55S. These were tested in the *in vitro* dissolution test, and the results are presented in Fig. 3. A conventional bicalutamide tablet formulation was included for comparison.

As Fig. 3 shows, for all of the formulations comprising HP-55S, 100% of bicalutamide in solution was achieved and supersaturation was maintained over the 60 minute test. These results were superior to the results achieved with the conventional formulation.

***In Vivo* Evaluation**

Oral doses of bicalutamide were administered to fasted dogs (equivalent to 450mg drug)(n=6). The formulations dosed were conventional CASODEX™ tablets and a 1:3 [bicalutamide:HP55S] solid dispersion. The solid dispersion was prepared as described earlier, however the solvent was removed by spray drying as opposed to rotary evaporation. Each oral dose was followed by 20ml of water. Blood samples were taken pre-dose and post dose at 1, 2, 3, 4, 6, 8, 12, 18, 24, 30, 36, 48, 72, 96, 120, 144, 168 hours. The samples centrifuged at 3000rpm for 15 minutes, the plasma removed into plain blood tubes and stored at -20°C until analysis. Samples were analysed by using a suitable extraction method followed by LC-MS.

Summary of Pharmacokinetic Parameters

| FORMULATION | Cpmax (µg/ml) | Tmax (hours) | AUC (µg/h/ml)* |
|---|---|---|---|
| HP-55S solid dispersion | 13 | 30 | 1504 ± 309 |
| Conventional formulation | 5 | 30 | 500 ± 405 |

* AUC from 0 to 144 hours

These data, as well as Fig. 4, show that the bioavailability of bicalutamide is greater with the solid dispersion using the enteric HP-55S polymer. In fact, the AUC measurements show a figure for the HP-55S solid dispersion that is almost 3 times that of the conventional tablet formulation. In addition, $C_{max}$ for the HP-55S solid dispersion is almost 3 times that of the conventional tablet formulation. Furthermore, inter-subject variability in the plasma levels of bicalutamide is lower with the HP-55S solid dispersion than with the conventional tablet formulation (for variability/total AUC, compare a figure of 309/1504 µg/h/ml for theHP-55S solid dispersion against a figure of 405/500 µg/h/ml for the conventional tablet formulation).

## Claims

1. A pharmaceutical formulation for mucosal administration to a patient, the formulation comprising bicalutamide in solid dispersion with an enteric polymer having a $pK_a$ from 3 to 6.

2. The formulation of claim 1, wherein the enteric polymer is selected from hydroxypropyl methylcellulose acetate succinate (HPMCAS), hydroxpropyl methylcellulose acetate pthalate, hydroxypropyl methylcellulose acetate, hydroxypropyl methylcellulose succinate a methacrylic acid copolymer, polyvinyl acetate phthalate (PVAP), cellulose acetate phthalate (CAP), methylcellulose acetate phthalate, ethyl cellulose acetate phthalate, hydroxypropyl cellulose acetate phthalate, hydroxypropyl methylcellulose phthalate (HPMCP), cellulose proprionate pthalate, hydroxypropyl cellulose butyrate pthalate, hydroxypropyl cellulose acetate pthalate succinate, hydroxypropyl methylcellulose trimellitate, cellulose acetate trimellitate (CAT), methylcellulose acetate trimellitate, ethyl cellulose acetate trimellitate, hydroxypropyl cellulose acetate trimellitate, hydroxypropyl methylcellulose acetate trimellitate, hydroxypropyl cellulose acetate trimellitate succinate, cellulose proprionate trimellitate, cellulose butyrate trimellitate, cellulose acetate terepthalate and cellulose actetate isopthalate.

3. The formulation of claim 2, wherein the enteric polymer is selected from HPMCP grade HP-50, HPMCP grade HP-55, HPMCP grade HP-55S, HPMCAS grade AS-LF, HPMCAS grade AS-MF, HPMCAS grade AS-HF, HPMCAS grade AS-LG, HPMCAS grade AS-MG, HPMCAS grade AS-HG, methacrylic acid copolymer grade A and methacrylic acid copolymer grade B.

4. The formulation of claim 3, wherein the enteric polymer is selected from HPMCP grade HP-55S, HPMCAS grade AS-LG and methacrylic acid copolymer grade A.

5. The formulation of any preceding claim, wherein the weight ratio of bicalutamide : enteric polymer is from 1:0.25 to 1:10.

6. The formulation of any preceding claim, wherein the solid dispersion comprises a wetting agent.

7. A daily pharmaceutical dose of bicalutamide mucosally administrable to a patient for treating and/or reducing the risk of prostate cancer in the patient, wherein the dose comprises from 25 to 1000 mg of bicalutamide in a solid

dispersion with an enteric polymer having a $pK_a$ from 3 to 6.

**8.** The daily dose of claim 7, wherein the enteric polymer is as defined in any one of claims 2 to 4.

**9.** The daily dose of claim 7 or 8, wherein the weight ratio of bicalutamide : enteric polymer is from 1:0.25 to 1:10.

**10.** The daily dose of any one of claims 7 to 9, wherein the solid dispersion comprises a wetting agent.

**11.** A solid dispersion of an enteric polymer having a $pK_a$ from 3 to 6 with bicalutamide for use as a medicament.

**12.** The solid dispersion of claim 11, wherein the enteric polymer is as defined in any one of claims 2 to 4.

**13.** The solid dispersion of claim 11 or 12, wherein the solid dispersion comprises a wetting agent.

**14.** Use of an enteric polymer having a $pK_a$ from 3 to 6 in solid dispersion with bicalutamide, in the manufacture of a medicament mucosally administrable to a patient, for increasing the bioavailability of bicalutamide in the patient.

**15.** Use of an enteric polymer having a $pK_a$ from 3 to 6 in solid dispersion with bicalutamide, in the manufacture of a medicament mucosally administrable to patients, for reducing inter-patient variability in plasma concentrations of bicalutamide.

**16.** Use of an enteric polymer having a $pK_a$ from 3 to 6 in solid dispersion with bicalutamide, in the manufacture of a medicament mucosally administrable to a patient, for treating and/or reducing the risk of prostate cancer in the patient.

**17.** The use according to any one of claims 14 to 16, wherein the medicament is provided as a daily dose of bicalutamide and comprises from 25 to 1000 mg of bicalutamide.

**18.** The use according to any one of claims 14 to 17, wherein the weight ratio of bicalutamide : enteric polymer is from 1:0.25 to 1:10.

**19.** The use according to any one of claims 14 to 18, wherein the solid dispersion includes a wetting agent.

## Patentansprüche

**1.** Pharmazeutische Formulierung zur mucosalen Verabreichung an einen Patienten, die Bicalutamid in fester Dispersion mit einem magensaftresistenten Polymer mit einem $pK_a$-Wert von 3 bis 6 enthält.

**2.** Formulierung nach Anspruch 1, bei der das magensaftresistente Polymer unter Hydroxypropylmethylcellulosesuccinat (HPMCAS), Hydroxypropylmethylcelluloseacetatphthalat, Hydroxypropylmethylcelluloseacetat, Hydroxypropylmethylcellulosesuccinat, einem Methacrylsäure-Copolymer, Polyvinylacetatphthalat (PVAP), Celluloseacetatphthalat(CAP), Methylcelluloseacetatphthalat, Ethylcelluloseacetatphthalat, Hydroxypropylcelluloseacetatphthalat, Hydroxypropylmethylcellulosephthalat (HPMCP), Cellulosepropionatphthalat, Hydroxypropylcellulosebutyratphthalat, Hydroxypropylcelluloseacetatphthalatsuccinat, Hydroxypropylmethylcellulosetrimellitat, Celluloseacetattrimellitat (CAT), Methylcelluloseacetattrimellitat, Ethylcelluloseacetattrimellitat, Hydroxypropylcelluloseacetattrimellitat, Hydroxypropylmethylcelluloseacetattrimellitat, Hydroxypropylcelluloseacetattrimellitatsuccinat, Cellulosepropionattrimellitat, Cellulosebutyrattrimellitat, Celluloseacetatterephthalat und Celluloseacetatisophthalat ausgewählt ist.

**3.** Formulierung nach Anspruch 2, bei der das magensaftresistente Polymer unter HPMCP Qualität HP-50, HPMCP Qualität HP-55, HPMCP Qualität HP-55S, HPMCAS Qualität AS-LF, HPMCAS Qualität AS-MF, HPMCAS Qualität AS-HF, HPMCAS Qualität AS-LG, HPMCAS Qualität AS-MG, HPMCAS Qualität AS-HG, Methacrylsäure-Copolymer Qualität A und Methacrylsäure-Copolymer Qualität B ausgewählt ist.

**4.** Formulierung nach Anspruch 3, bei der das magensaftresistente Polymer unter HPMCP Qualität HP-55S, HPMCAS Qualität AS-LG und Methacrylsäure-Copolymer Qualität A ausgewählt ist.

**5.** Formulierung nach einem der vorhergehenden Ansprüche, bei der das Gewichtsverhältnis von Bicalutamid zu magensaftresistentem Polymer 1:0,25 bis 1:10 beträgt.

**6.** Formulierung nach einem der vorhergehenden Ansprüche, bei der die feste Dispersion ein Netzmittel enthält.

**7.** An einen Patienten zur Behandlung und/oder Verringerung des Risikos von Prostatakrebs bei dem Patienten mucosal verabreichbare pharmazeutische Tagesdosis von Bicalutamid, die 25 bis 1000 mg Bicalutamid in fester Dispersion mit einem magensaftresistenten Polymer mit einem $pK_a$-Wert von 3 bis 6 enthält.

**8.** Tagesdosis nach Anspruch 7, bei der das magensaftresistente Polymer wie in einem der Ansprüche 2 bis 4 definiert ist.

**9.** Tagesdosis nach Anspruch 7 oder 8, bei der das Gewichtsverhältnis von Bicalutamid zu magensaftresistentem Polymer 1:0,25 bis 1:10 beträgt.

**10.** Tagesdosis nach einem der Ansprüche 7 bis 9, bei der die feste Dispersion ein Netzmittel enthält.

**11.** Feste Dispersion eines magensaftresistenten Polymers mit einem $pK_a$-Wert von 3 bis 6 mit Bicalutamid zur Verwendung als Arzneimittel.

**12.** Feste Dispersion nach Anspruch 11, bei der das magensaftresistente Polymer wie in einem der Ansprüche 2 bis 4 definiert ist.

**13.** Feste Dispersion nach Anspruch 11 oder 12, bei der die feste Dispersion ein Netzmittel enthält.

**14.** Verwendung eines magensaftresistenten Polymers mit einem $pK_a$-Wert von 3 bis 6 in fester Dispersion mit Bicalutamid bei der Herstellung eines an einen Patienten mucosal verabreichbaren Arzneimittels zur Erhöhung der Bioverfügbarkeit von Bicalutamid bei dem Patienten.

**15.** Verwendung eines magensaftresistenten Polymers mit einem $pK_a$-Wert von 3 bis 6 in fester Dispersion mit Bicalutamid bei der Herstellung eines an Patienten mucosal verabreichbaren Arzneimittels zur Verringerung der Variabilität der Plasmakonzentrationen von Bicalutamid zwischen verschiedenen Patienten.

**16.** Verwendung eines magensaftresistenten Polymers mit einem $pK_a$-Wert von 3 bis 6 in fester Dispersion mit Bicalutamid bei der Herstellung eines an Patienten mucosal verabreichbaren Arzneimittels zur Behandlung und/oder Verringerung des Risikos von Prostatakrebs bei dem Patienten.

**17.** Verwendung nach einem der Ansprüche 14 bis 16, bei der das Arzneimittel als Tagesdosis von Bicalutamid bereitgestellt wird und 25 bis 1000 mg Bicalutamid enthält.

**18.** Verwendung nach einem der Ansprüche 14 bis 17, bei der das Gewichtsverhältnis von Bicalutamid zu magensaftresistentem Polymer 1:0,25 bis 1:10 beträgt.

**19.** Verwendung nach einem der Ansprüche 14 bis 18, bei der die feste Dispersion ein Netzmittel enthält.

## Revendications

**1.** Formulation pharmaceutique destinée à une administration par voie muqueuse à un patient, la formulation comprenant du bicalutamide en dispersion solide avec un polymère entérique présentant un $pK_a$ de 3 à 6.

**2.** Formulation selon la revendication 1, dans laquelle le polymère entérique est choisi parmi l'acétate-succinate d'hydroxypropylméthylcellulose (HPMCAS), l'acétate-phtalate d'hydroxypropylméthylcellulose, l'acétate d'hydroxypropylméthylcellulose, le succinate d'hydroxypropylméthylcellulose, un copolymère d'acide méthacrylique, l'acétate-phtalate de polyvinyle (PVAP), l'acétate-phtalate de cellulose (CAP), l'acétate-phtalate de méthylcellulose, l'acétate-phtalate d'éthylcellulose, l'acétate-phtalate d'hydroxypropylcellulose, le phtalate d'hydroxypropylméthylcellulose (HPMCP), le propionate-phtalate de cellulose, le butyrate-phtalate d'hydroxypropylcellulose, l'acétate-phtalate-succinate d'hydroxypropylcellulose, le trimellitate d'hydroxypropylméthylcellulose, l'acétate-trimellitate

de cellulose (CAT), l'acétate-trimellitate de méthylcellulose, l'acétate-trimellitate d'éthylcellulose, l'acétate-trimellitate d'hydroxypropylcellulose, l'acétate-trimellitate d'hydroxypropylméthylcellulose, l'acétate-trimellitate-succinate d'hydroxypropylcellulose, le proprionate-trimellitate de cellulose, le butyrate-trimellitate de cellulose, l'acétate-téréphtalate de cellulose et l'acétate-isophtalate de cellulose.

**3.** Formulation selon la revendication 2, dans laquelle le polymère entérique est choisi parmi le HPMCP de qualité HP-50, le HPMCP de qualité HP-55, le HPMCP de qualité HP-55S, le HPMCAS de qualité AS-LF, le HPMCAS de qualité AS-MF, le HPMCAS de qualité AS-HF, le HPMCAS de qualité AS-LG, le HPMCAS de qualité AS-MG, le HPMCAS de qualité AS-HG, un copolymère d'acide méthacrylique de qualité A et un copolymère d'acide méthméthacrylique de qualité B.

**4.** Formulation selon la revendication 3, dans laquelle le polymère entérique est choisi parmi le HPMCP de qualité HP-55S, le HPMCAS de qualité AS-LG et un copolymère d'acide méthacrylique de qualité A.

**5.** Formulation selon l'une quelconque des revendications précédentes, dans laquelle le rapport pondéral du bicalutamide : polymère entérique est de 1:0,25 à 1:10.

**6.** Formulation selon l'une quelconque des revendications précédentes, dans laquelle la dispersion solide comprend un agent mouillant.

**7.** Dose pharmaceutique journalière de bicalutamide administrable par voie muqueuse à un patient pour le traitement et/ou la réduction du risque d'un cancer de la prostate chez le patient, la dose comprenant de 25 à 1000 mg de bicalutamide dans une dispersion solide avec un polymère entérique présentant un $pK_a$ de 3 à 6.

**8.** Dose journalière selon la revendication 7, dans laquelle le polymère entérique est tel que défini dans l'une quelconque des revendications 2 à 4.

**9.** Dose journalière selon la revendication 7 ou 8, dans laquelle le rapport pondéral du bicalutamide:polymère entérique est de 1:0,25 à 1:10.

**10.** Dose journalière selon l'une quelconque des revendications 7 à 9, dans laquelle la dispersion solide comprend un agent mouillant.

**11.** Dispersion solide d'un polymère entérique présentant un $pK_a$ de 3 à 6 avec du bicalutamide, destinée à être utilisée en tant que médicament.

**12.** Dispersion solide selon la revendication 11, dans laquelle le polymère entérique est tel que défini dans l'une quelconque des revendications 2 à 4.

**13.** Dispersion solide selon la revendication 11 ou 12, la dispersion solide comprenant un agent mouillant.

**14.** Utilisation d'un polymère entérique présentant un $pK_a$ de 3 à 6 en dispersion solide avec du bicalutamide, pour la fabrication d'un médicament administrable par voie muqueuse à un patient, pour accroître la biodisponibilité du bicalutamide chez le patient.

**15.** Utilisation d'un polymère entérique présentant un $pK_a$ de 3 à 6 en dispersion solide avec du bicalutamide, pour la fabrication d'un médicament administrable par voie muqueuse à des patients, pour la réduction de la variabilité entre les patients dans des concentrations plasmatiques de bicalutamide.

**16.** Utilisation d'un polymère entérique présentant un $pK_a$ de 3 à 6 en dispersion solide avec du bicalutamide, pour la fabrication d'un médicament administrable par voie muqueuse à un patient, pour le traitement et/ou la réduction du risque d'un cancer de la prostate chez le patient.

**17.** Utilisation selon l'une quelconque des revendications 14 à 16, dans laquelle le médicament est procuré sous forme d'une dose journalière de bicalutamide et comprend de 25 à 1000 mg de bicalutamide.

**18.** Utilisation selon l'une quelconque des revendications 14 à 17, dans laquelle le rapport pondéral du bicalutamide: polymère entérique est de 1:0,25 à 1:10.

**19.** Utilisation selon l'une quelconque des revendications 14 à 18, dans laquelle la dispersion solide comprend un agent mouillant.

Fig.1

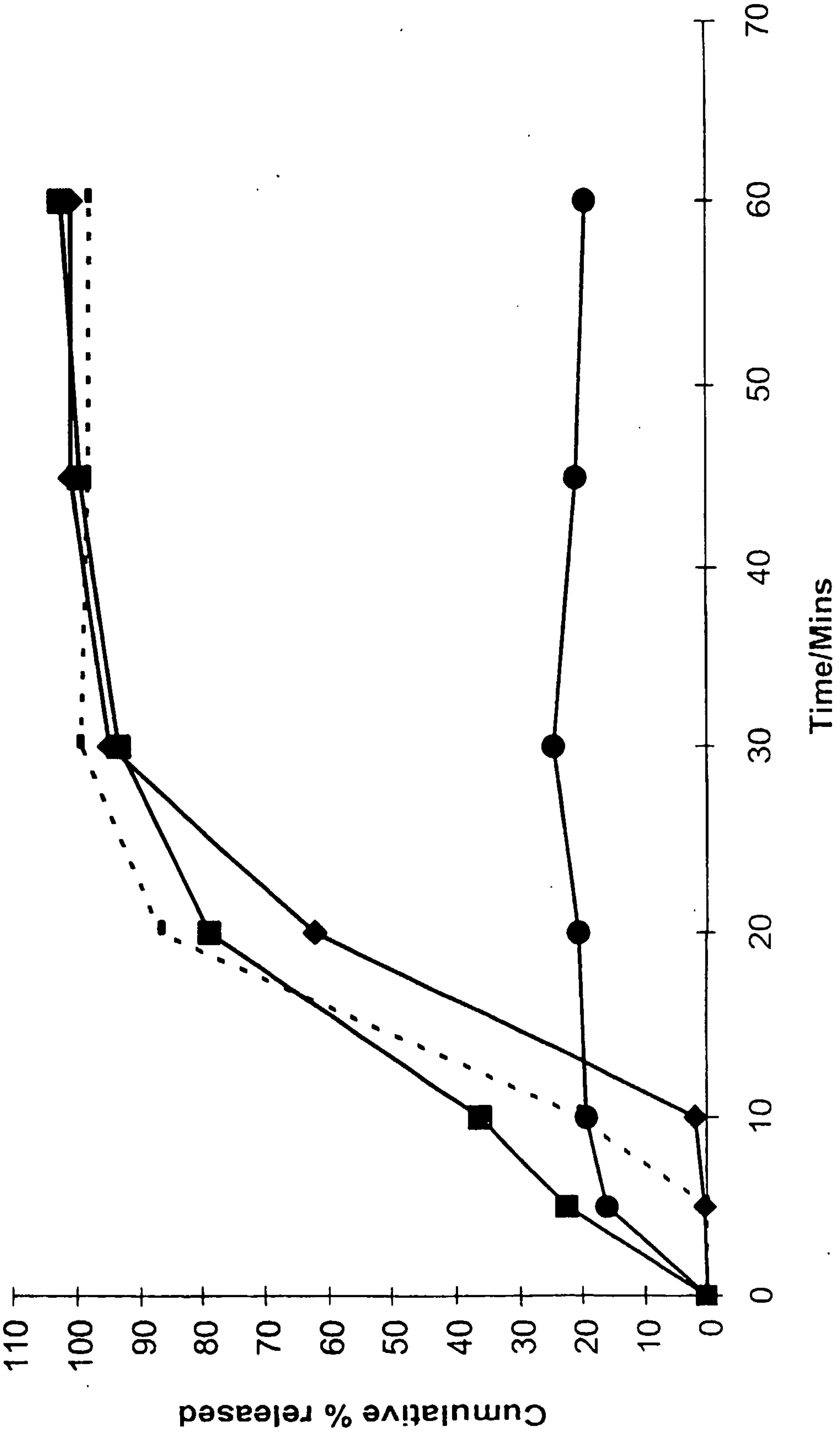
Cumulative % released
110
100
90
80
70
60
50
40
30
20
10
0
0
10
20
30
40
50
60
70
Time/Mins

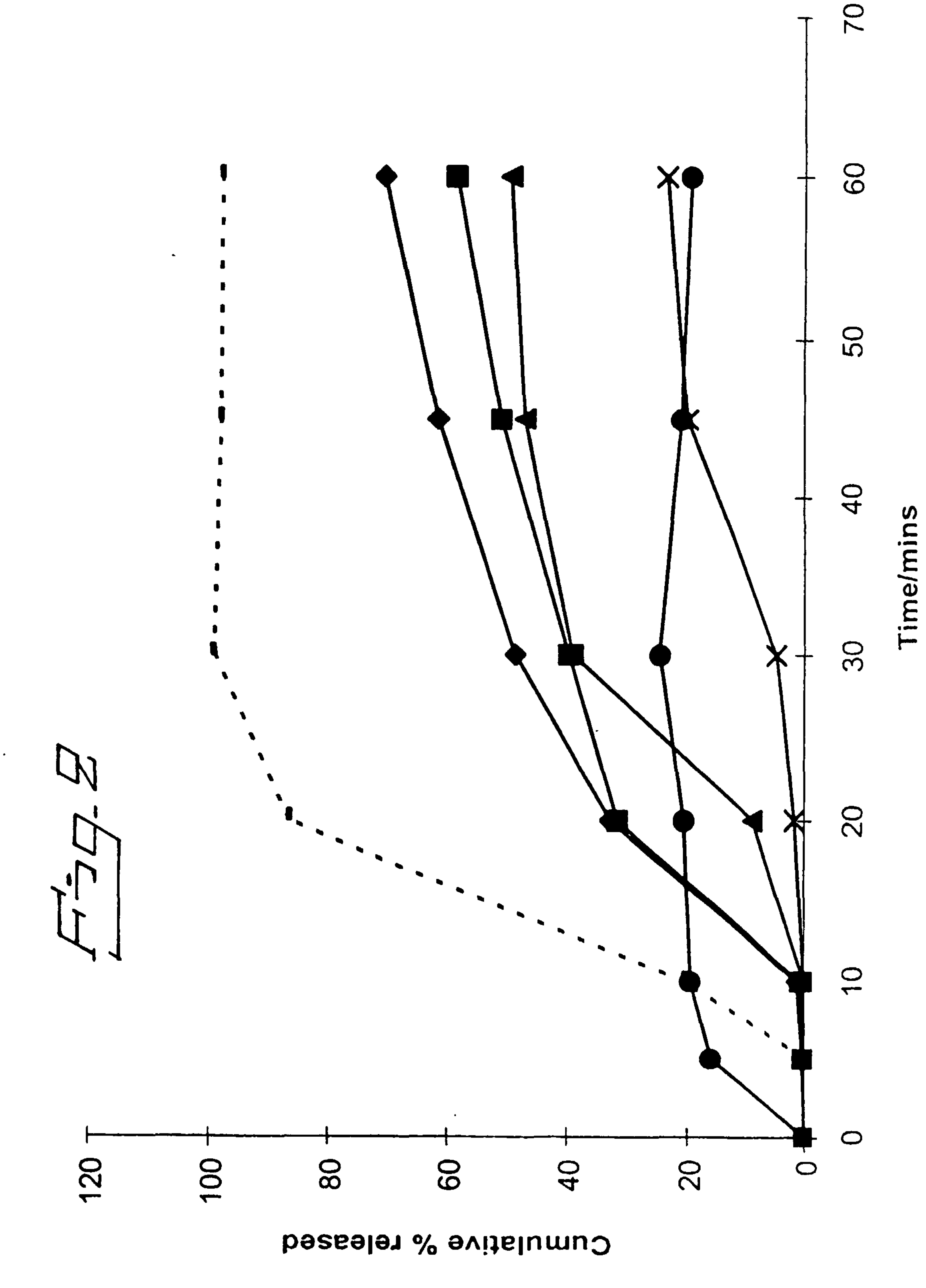
Cumulative % released
0
20
40
60
80
100
120
Time/mins
0
10
20
30
40
50
60
70

Fig. 2

Fig. 3

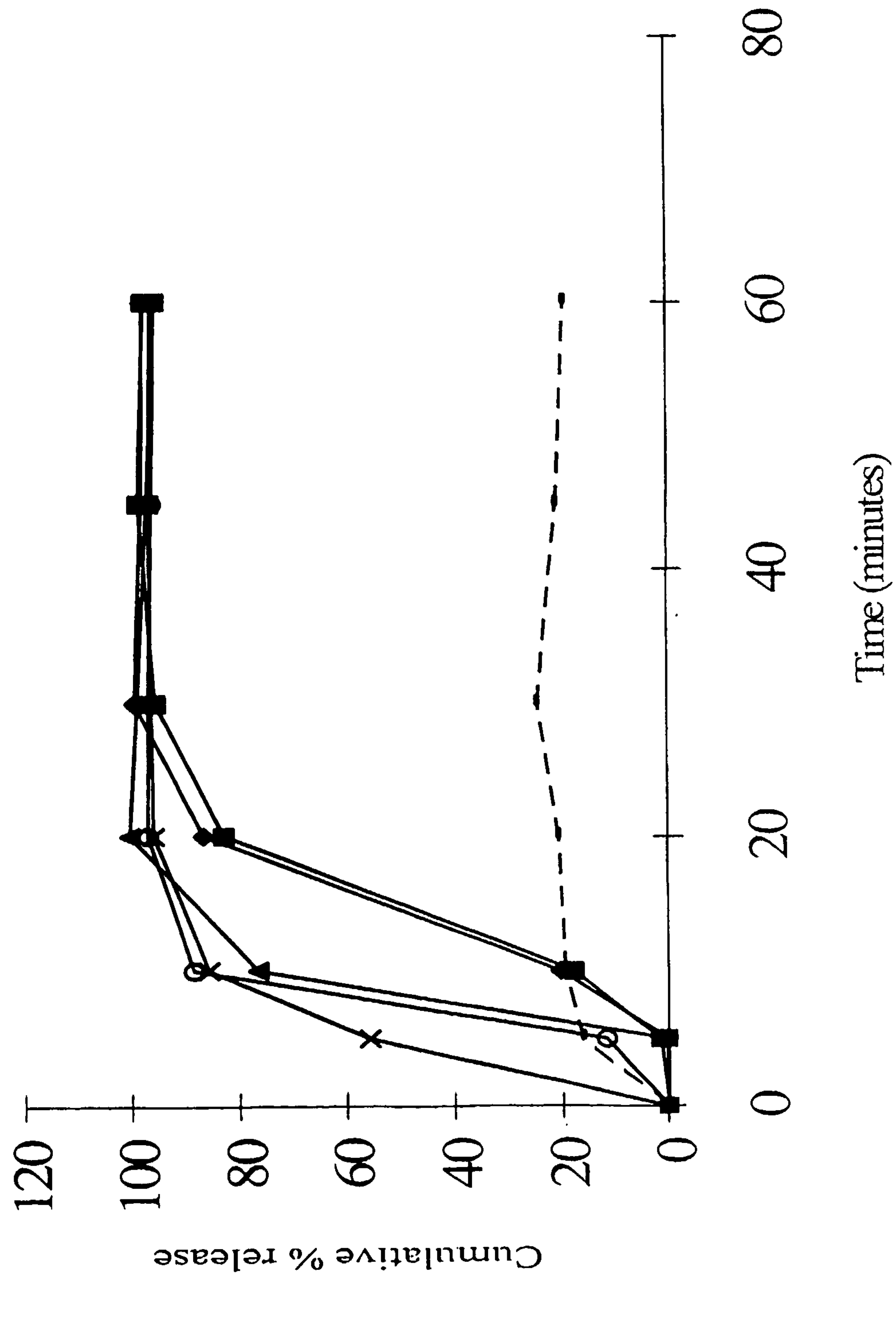
Cumulative % release
0
20
40
60
80
100
120
Time (minutes)
0
20
40
60
80

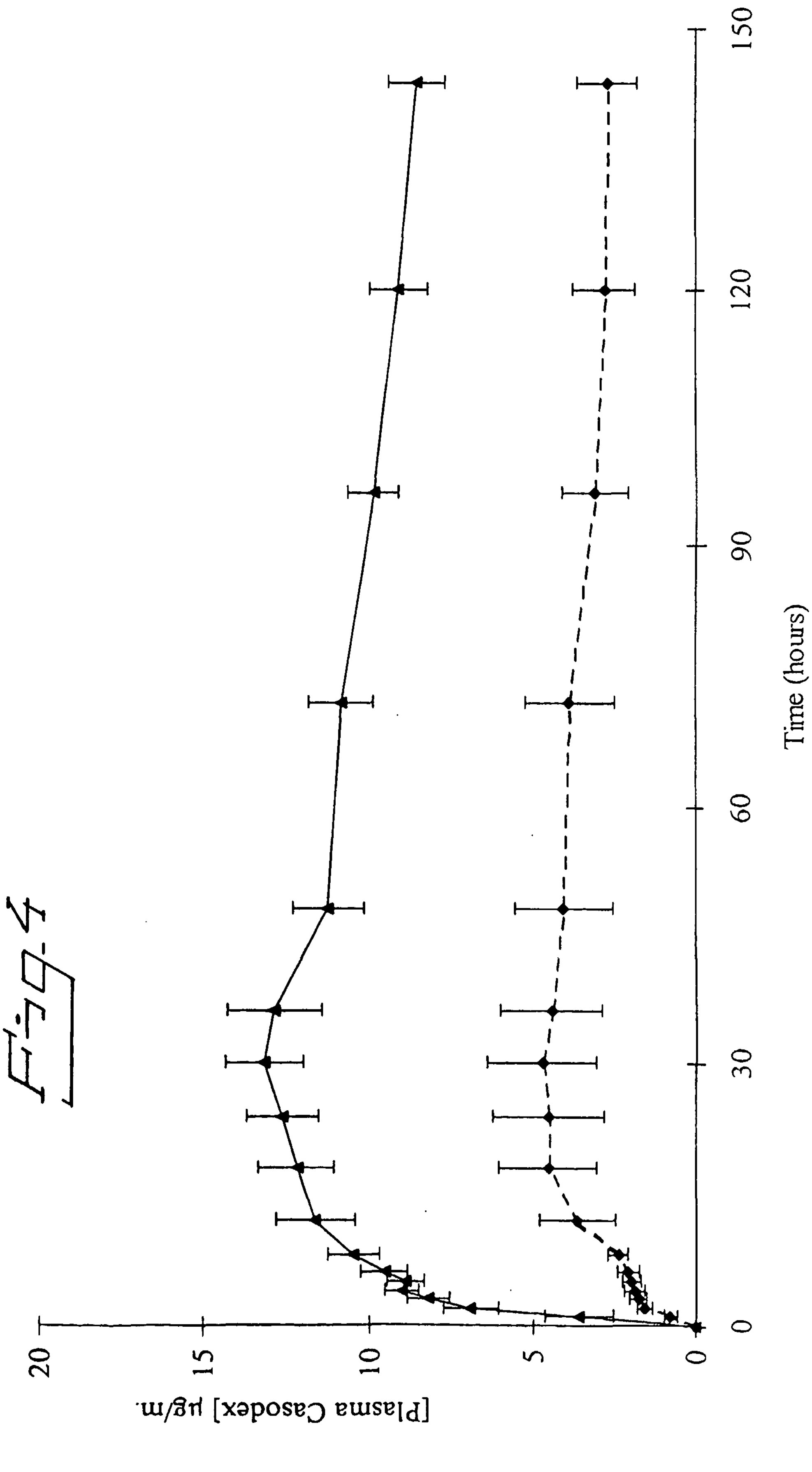


Fig. 4